# EUROPEAN PATENT APPLICATION

(11) **EP 4 389 727 A1**
(43) Date of publication of application: **26.06.2024**
(21) Application number: 22215170.6
(22) Date of filing: 20.12.2022
(51) Int. Cl.: C07C 4/22, C08J 11/04

(54) **PROCESS FOR RECOVERY OF STYRENE MONOMER FROM WASTE POLYSTYRENE**

(71) Applicant: SABIC Global Technologies B.V., 4612 PX Bergen op Zoom (NL)
(72) Inventor: Schucker, Robert C., 562125 Bengaluru (IN)
(74) Representative: Sabic INDIA Intellectual Property Group

(57) **Abstract**

A method of processing polystyrene is disclosed. The method includes contacting polystyrene with an aromatic solvent at a temperature in a range of 325 to 425 °C and a pressure in a range of 50 to 300 torr to produce a gas comprising styrene monomer. The method further includes separating the styrene monomer from one or more components of the gas to form a product stream comprising styrene.

## Description

### CROSS REFERENCE TO RELATED APPLICATIONS

None.

### FIELD OF INVENTION

The present invention generally relates to processing of waste polystyrene. More specifically, the present invention relates to processing of waste polystyrene to produce styrene monomer.

### BACKGROUND OF THE INVENTION

Expanded polystyrene foam is used in a variety of consumer products. For example, polystyrene foam is used as food containers and hot drink cups. These consumer items are particularly difficult to collect and recycle because of their light weight (densities range from 0.1 - 0.3 gm/cc). While polystyrene foam can be mechanically recycled, such recycling downgrades its value. And chemical recycling of polystyrene by contemporary commercial processes is energy intensive and inefficient.

### BRIEF SUMMARY OF THE INVENTION

The present inventor has discovered a selective process for depolymerizing polystyrene to form styrene monomer, which can be used to resynthesize the original polystyrene polymer or be converted to other chemicals that, in turn, can be used to manufacture polymers such as acrylonitrile-butadiene-styrene (ABS). The discovered process involves the use of a solvent, which can enhance heat transfer and mass transfer and incorporates the use of a slight vacuum; all of which allows the process to occur at an overall lower temperature than contemporary processes, thereby saving energy. Moreover, the discovered process does not require a catalyst (although in embodiments of the invention one or more catalysts could be used).

Embodiments of the invention include a method of processing polystyrene. The method includes contacting polystyrene with an aromatic solvent at a temperature in a range of 325 to 425 °C and a pressure in a range of 50 to 400 torr to produce a gas comprising styrene monomer. The method further includes separating the styrene monomer from one or more components of the gas to form a product stream comprising styrene.

Embodiments of the invention include a method of processing polystyrene. The method includes processing polystyrene bricks to produce polystyrene particles. The method further includes mixing, in a reactor, the polystyrene particles, a polymerization inhibitor (optionally), and a highly aromatic, high boiling point solvent, at a temperature in a range of 325 to 425 °C and a pressure in a range of 50 to 400 torr to produce a gas product comprising styrene, ethylbenzene, toluene, and benzene. Further yet, the method includes flowing the gas from the reactor to a first vacuum distillation column and distilling the gas in the first vacuum distillation column to produce a first stream comprising primarily toluene and benzene and a second stream comprising primarily styrene and ethylbenzene. The method also includes flowing the second stream to a second vacuum distillation column and distilling the second stream in the second vacuum distillation column to produce a third stream comprising primarily ethylbenzene and a fourth stream comprising primarily styrene.

The following includes definitions of various terms and phrases used throughout this specification.

The terms "about" or "approximately" are defined as being close to as understood by one of ordinary skill in the art. In one non-limiting embodiment the terms are defined to be within 10%, preferably, within 5%, more preferably, within 1%, and most preferably, within 0.5%.

For the purposes of this disclosure, "X, Y, and/or Z" can be construed as X only, Y only, Z only, or any combination of two or more items X, Y, and Z (e.g., XYZ, XY, XZ, YZ).

The terms "wt.%", "vol.%" or "mol.%" refer to a weight, volume, or molar percentage of a component, respectively, based on the total weight, the total volume, or the total moles of material that includes the component. In a non-limiting example, 10 moles of component in 100 moles of the material is 10 mol.% of component.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification, include any measurable decrease or complete inhibition to achieve a desired result.

The term "effective," as that term is used in the specification and/or claims, means adequate to accomplish a desired, expected, or intended result.

The use of the words "a" or "an" when used in conjunction with the term "comprising," "including," "containing," or "having" in the claims or the specification may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The process of the present invention can "comprise," "consist essentially of," or "consist of" particular ingredients, components, compositions, etc., disclosed throughout the specification.

The term "primarily," as that term is used in the specification and/or claims, means greater than any of 50 wt.%, 50 mol.%, and 50 vol.%. For example, "primarily" may include 50.1 wt.% to 100 wt.% and all values and ranges there between, 50.1 mol.% to 100 mol.% and all values and ranges there between, or 50.1 vol.% to 100 vol.% and all values and ranges there between.

Other objects, features and advantages of the present invention will become apparent from the following figures, detailed description, and examples. It should be understood, however, that the figures, detailed description, and examples, while indicating specific embodiments of the invention, are given by way of illustration only and are not meant to be limiting. Additionally, it is contemplated that changes and modifications within the spirit and scope of the invention will become apparent to those skilled in the art from this detailed description. In further embodiments, features from specific embodiments may be combined with features from other embodiments. For example, features from one embodiment may be combined with features from any of the other embodiments. In further embodiments, additional features may be added to the specific embodiments described herein.

### BRIEF DESCRIPTION OF THE DRAWINGS

For a more complete understanding, reference is now made to the following descriptions taken in conjunction with the accompanying drawings, in which:
FIG. 1 shows a system for processing polystyrene, according to embodiments of the invention; and
FIG. 2 shows a method of processing polystyrene, according to embodiments of the invention.

### DETAILED DESCRIPTION OF THE INVENTION

The present inventor has discovered a method for processing polystyrene that uses a readily available, cheap, highly aromatic solvent as a process fluid that facilitates thermal decomposition of polystyrene, in a reactor, into volatile liquids that can be removed from the reactor using a light vacuum. The use of a solvent enhances heat transfer and mass transfer and allows the process to occur at an overall lower temperature than contemporary processes-thereby being more energy efficient than contemporary processes. Embodiments of the invention can also be carried out without a catalyst-thereby avoiding the cost of catalytic materials.

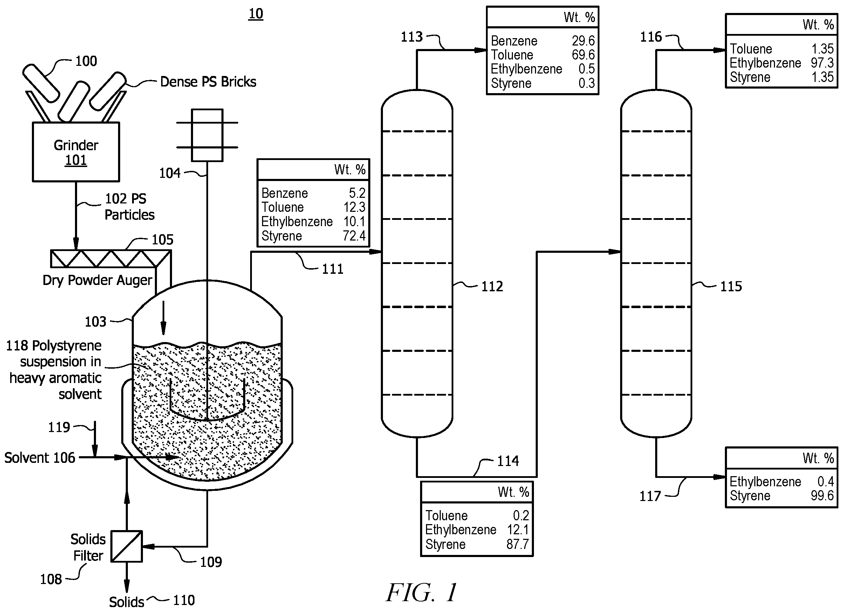

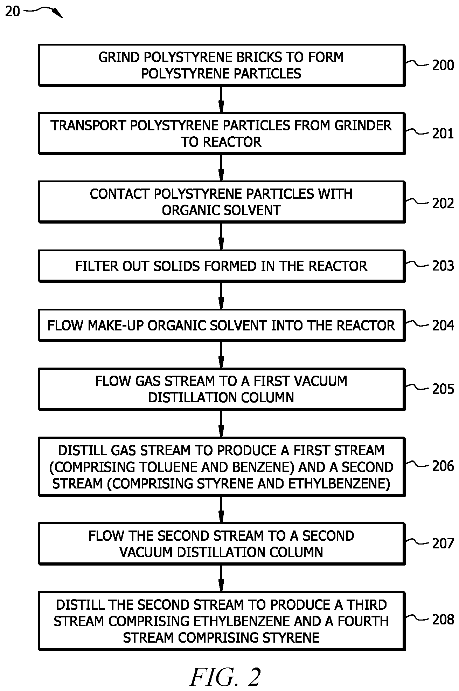

FIG. 1 shows system 10 for processing polystyrene, according to embodiments of the invention. FIG. 2 shows method 20 for processing polystyrene, according embodiments of the invention. In embodiments of the invention, method 20 can be implemented using system 10.

### System for processing polystyrene

According to embodiments of the invention, system 10 comprises grinder 101, which is adapted to grind polystyrene bricks 100 to form polystyrene particles 102, which may have a range of particle sizes. System 10 further includes dry powder auger 105, which is adapted to transport polystyrene particles 102 from grinder 101 to reactor 103, according to embodiments of the invention. In embodiments of the invention, reactor 103 can be a continuous flow stirred tank reactor (CSTR), which is relatively cheap and can easily be operated under a slight vacuum. Reactor 103, in embodiments of the invention, comprises agitator 104 for agitating contents of reactor 103-contents such as polystyrene particles 102 and aromatic solvent 106. In embodiments of the invention, aromatic solvent 106 comprises a high boiling point aromatic solvent such as light cat cycle oil (LCO), heavy cat cycle oil (HCCO), preferably lube extract, or any other suitable aromatic solvent. According to embodiments of the invention, reactor 103 is adapted to heat polystyrene suspension in heavy aromatic solvent 118 under a vacuum so as to decompose polystyrene and produce gas stream 111. Reactor 103, in embodiments of the invention, is in fluid communication with first vacuum distillation column 112 such that gas stream 111 can flow from reactor 103 to first vacuum distillation column 112. In embodiments of the invention, gas stream 111 comprises styrene, ethylbenzene, toluene, and benzene. According to embodiments of the invention, first vacuum distillation column 112 is adapted to separate gas stream 111 into first stream 113, comprising primarily toluene and benzene, second stream 114, comprising primarily styrene and ethylbenzene. In embodiments of the invention, second vacuum distillation column 115 is adapted to receive and distill second stream 114 to produce third stream 116, comprising primarily ethylbenzene and fourth stream 117 comprising primarily styrene. System 10, in embodiments of the invention, also includes solids filter 108, which is adapted to filter solids 110 from recycle stream 109.

### Method for processing polystyrene

According to embodiments of the invention, method 20 comprises, at block 200, grinder 101 grinding polystyrene material, such as polystyrene bricks 100, to form polystyrene particles 102. Block 201, of method 20, in embodiments of the invention, involves dry powder auger 105 transporting polystyrene particles 102 from grinder 101 to reactor 103. According to embodiments of the invention, at block 202, method 20 involves contacting polystyrene particles 102 with aromatic solvent 106. Aromatic solvent 106 can comprise light cat cycle oil, heavy cat cycle oil, lube extract, or any other suitable aromatic solvent; and aromatic solvent 106 can be disposed in reactor 103 prior to flowing polystyrene particles 102 into reactor 103. The contacting at block 202, in embodiments of the invention, can be enhanced by agitator 104 mixing polystyrene particles 102 and aromatic solvent 106 in reactor 103 to form polystyrene suspension in heavy aromatic solvent 118. The concentration of polystyrene in aromatic solvent, in embodiments of the invention, is 5-20 wt.% with processing rates of 1-10 KTA. According to embodiments of the invention, the contacting of polystyrene particles 102 and aromatic solvent 106, in reactor 103, at suitable reaction conditions, decomposes polystyrene particles 102 to form gas stream 111, comprising styrene, toluene, ethylbenzene, and benzene. The reaction conditions, in embodiments of the invention, for carrying out the polystyrene decomposition process, include a temperature in a range of 325 to 425 °C, including ranges of 325 to 335 °C, 335 to 345 °C, 345 to 355 °C, 355 to 365 °C, 365 to 375 °C, 375 to 385 °C, 385 to 395 °C, 395 to 405 °C, 405 to 415 °C, and 415 to 425 °C. External heating may be used to maintain reaction conditions in the desired temperature range, in embodiments of the invention. The reaction conditions, in embodiments of the invention, for carrying out the polystyrene decomposition process, include having reactor 103 under vacuum such that there is a pressure in a range of 50 to 400 torr, including ranges of 50 to 75 torr, 75 to 100 torr, 100 to 125 torr, 125 to 150 torr, 150 to 175 torr, 175 to 200 torr, 200 to 225 torr, 225 to 250 torr, 250 to 275 torr, 275 to 300 torr, 300 to 325 torr, 325 to 350 torr, 350 to 375 torr, 375 to 400 torr.

In embodiments of the invention, to minimize the risks associated with undesired polymerization, a polymerization inhibitor, polymerization inhibitor 119, such as tert-butylcatechol (TBC, bp 285 °C) can be added to aromatic solvent 106, in reactor 103, and thus polystyrene suspension in heavy aromatic solvent 118 can be comprised of aromatic solvent 106, polystyrene particles 102, and polymerization inhibitor 119. In other words, in embodiments of the invention, block 202 can include contacting aromatic solvent 106, polystyrene particles 102, and polymerization inhibitor 119 with each other. According to embodiments of the invention, block 203 involves flowing recycle stream 109 through filter 108, which filters out solids 110 formed in reactor 103. Filter 108, according to embodiments of the invention, is adapted with the right sieve size to remove solids 110. If any of aromatic solvent 106 is lost overhead, at block 204, make-up aromatic solvent 106 is flowed into reactor 103, in embodiments of the invention.

According to embodiments of the invention, block 205 includes flowing gas stream 111 to first vacuum distillation column 112. And at block 206, in embodiments of the invention, first vacuum distillation column 112 distills gas stream 111 to produce first stream 113 (comprising primarily toluene and benzene) and second stream 114 (comprising primarily styrene and ethylbenzene). In embodiments of the invention, gas stream 111 comprises 69 to 75 wt.% styrene, 10 to 15 wt.% toluene, 8 to 12 wt.% ethylbenzene, 2 to 7 wt.% benzene. In embodiments of the invention, first stream 113 comprises 0.1 to 1.0 wt.% styrene, 65 to 75 wt.% toluene, 0.1 to 1.5 wt.% ethylbenzene, 27 to 33 wt.% benzene. In embodiments of the invention, second stream 114 comprises 82 to 92 wt.% styrene, 0.1 to 1.0 wt.% toluene, and 10 to 15 wt.% ethylbenzene.

In embodiments of the invention, block 207 includes flowing second stream 114 to second vacuum distillation column 115. And at block 208, in embodiments of the invention, second vacuum distillation column 115 distills second stream 114 to produce third stream 116 (comprising primarily ethylbenzene) and fourth stream 117 (comprising primarily styrene). In embodiments of the invention, third stream 116 comprises 1.0 to 2.0 wt.% styrene, 1.0 to 2.0 wt.% toluene, and 95 to 99.9 wt.% ethylbenzene. In embodiments of the invention, fourth stream 117 comprises 99 to 99.9 wt. % styrene, and 0.1 to 1 wt. % ethylbenzene. As noted above, in embodiments of the invention, reactor 103 is operated under vacuum and the separation process is carried out by first vacuum distillation column 112 and second vacuum distillation column 115, all of which helps in carrying out the distillation separation at lower temperatures than conventional methods and thereby circumventing the possibilities of runaway polymerization between components.

Although embodiments of the present invention have been described with reference to blocks of FIG. 2, it should be appreciated that operation of the present invention is not limited to the particular blocks and/or the particular order of the blocks illustrated in FIG. 2. Accordingly, embodiments of the invention may provide functionality as described herein using various blocks in a sequence different than that of FIG. 2.

The systems and processes described herein can also include various equipment that is not shown and is known to one of skill in the art of chemical processing. For example, some controllers, piping, computers, valves, pumps, heaters, thermocouples, pressure indicators, mixers, heat exchangers, and the like may not be shown.

In the context of the present invention, at least the following 15 embodiments are described. Embodiment 1 is a method of processing polystyrene. The method includes contacting polystyrene with an aromatic solvent at a temperature in a range of 325 to 425 °C and a pressure in a range of 50 to 400 torr to produce a gas comprising styrene monomer. The method further includes separating the styrene monomer from one or more components of the gas to form a product stream comprising styrene. Embodiment 2 is the method of embodiment 1 further comprising processing the styrene monomer to produce a polymer. Embodiment 3 is the method of embodiment 2, wherein the polymer comprises polystyrene and acrylonitrile-butadiene-styrene (ABS). Embodiment 4 is the method of any of embodiments 1 to 3, further comprising grinding polystyrene bricks to form polystyrene particles, wherein the polystyrene particles are used for the contacting of the polystyrene with the aromatic solvent. Embodiment 5 is the method of embodiment 4, wherein the polystyrene bricks are derived from polystyrene waste. Embodiment 6 is the method of any of embodiments 1 to 5, further comprising contacting a polymerization inhibitor with the polystyrene and aromatic solvent. Embodiment 7 is the method of embodiment 6, wherein the polymerization inhibitor comprises tert-butylcatechol. Embodiment 8 is the method of any of embodiments 1 to 7, further comprising carrying out the contacting of the polystyrene with the aromatic solvent in a reactor comprising an agitator adapted to mix contents of the reactor. Embodiment 9 is the method of embodiment 8, wherein the reactor comprises a continuous flow stirred tank reactor (CSTR). Embodiment 10 is the method of any of embodiments 1 to 9, wherein the separating of the gas is carried out by a first vacuum distillation column and a second vacuum distillation column. Embodiment 11 is the method of embodiment 10, wherein the first vacuum distillation column distills the gas to produce a first stream comprising primarily toluene and benzene and a second stream comprising primarily styrene and ethylbenzene. Embodiment 12 is the method of embodiment 11, wherein the second vacuum distillation column distills the second stream to produce a third stream comprising primarily ethylbenzene and the product stream comprising styrene. Embodiment 13 is the method of any of embodiments 1 to 12, wherein the aromatic solvent is selected from the list consisting of: light cat cycle oil (LCO), heavy cat cycle oil (HCCO), and lube extract. Embodiment 14 is the method of any of embodiments 1 to 13, wherein the product stream comprises 99 to 99.9 wt.% styrene and 0.1 to 1 wt.% ethylbenzene. Embodiment 15 is the method of any of embodiments 1 to 14, wherein the contacting of the polystyrene with the aromatic solvent occurs at a pressure in a range of 50 to 300 torr. Embodiment 16 is a method of processing polystyrene. The method comprises processing polystyrene bricks to produce polystyrene particles. The method further comprises mixing, in a reactor, the polystyrene particles, a polymerization inhibitor, and an aromatic solvent, at a temperature in a range of 325 to 425 °C and a pressure in a range of 50 to 400 torr to produce a gas comprising styrene, ethylbenzene, toluene, and benzene. The method still further comprises flowing the gas from the reactor to a first vacuum distillation column; distilling the gas in the first vacuum distillation column to produce a first stream comprising primarily toluene and benzene and a second stream comprising primarily styrene and ethylbenzene; flowing the second stream to a second vacuum distillation column; and distilling the second stream in the second vacuum distillation column to produce a third stream comprising primarily ethylbenzene and a fourth stream comprising primarily styrene.

## Claims

1. A method of processing polystyrene, the method comprising:
contacting polystyrene with an aromatic solvent at a temperature in a range of 325 to 425 °C and a pressure in a range of 50 to 400 torr to produce a gas comprising styrene monomer; and
separating the styrene monomer from one or more components of the gas to form a product stream comprising styrene.

2. The method of claim 1 further comprising:
processing the styrene monomer to produce a polymer.

3. The method of claim 2, wherein the polymer comprises polystyrene and acrylonitrile-butadiene-styrene (ABS).

4. The method of any of claims 1 to 3, further comprising:
grinding polystyrene bricks to form polystyrene particles, wherein the polystyrene particles are used for the contacting of the polystyrene with the aromatic solvent.

5. The method of claim 4, wherein the polystyrene bricks are derived from polystyrene waste.

6. The method of any of claims 1 to 5, further comprising:
contacting a polymerization inhibitor with the polystyrene and aromatic solvent.

7. The method of claim 6, wherein the polymerization inhibitor comprises tert-butylcatechol.

8. The method of any of claims 1 to 7, further comprising:
carrying out the contacting of the polystyrene with the aromatic solvent in a reactor comprising an agitator adapted to mix contents of the reactor.

9. The method of claim 8, wherein the reactor comprises a continuous flow stirred tank reactor (CSTR).

10. The method of any of claims 1 to 9, wherein the separating of the gas is carried out by a first vacuum distillation column and a second vacuum distillation column.

11. The method of claim 10, wherein the first vacuum distillation column distills the gas to produce a first stream comprising primarily toluene and benzene and a second stream comprising primarily styrene and ethylbenzene.

12. The method of claim 11, wherein the second vacuum distillation column distills the second stream to produce a third stream comprising primarily ethylbenzene and the product stream comprising styrene.

13. The method of any of claims 1 to 12, wherein the aromatic solvent is selected from the list consisting of: light cat cycle oil (LCO), heavy cat cycle oil (HCCO), and lube extract.

14. The method of any of claims 1 to 13, wherein the product stream comprises 99 to 99.9 wt.% styrene and 0.1 to 1 wt.% ethylbenzene.

15. The method of any of claims 1 to 14, wherein the contacting of the polystyrene with the aromatic solvent occurs at a pressure in a range of 50 to 300 torr.
